# EUROPEAN PATENT APPLICATION

(11) **EP 4 397 310 A1**
(43) Date of publication of application: **10.07.2024**
(21) Application number: 21955893.9
(22) Date of filing: 30.08.2021
(51) Int. Cl.: A61K 31/713, A61P 35/00, A61P 35/04

(54) **PHARMACEUTICAL COMPOSITION FOR SUPPRESSING INVASION OF CANCER, AND METHOD FOR SUPPRESSING INVASION OF CANCER**

(71) Applicant: TME Therapeutics Inc., Tokyo, 105-0021 (JP)
(72) Inventor: YONEYAMA Hiroyuki, Tokyo 105-0021 (JP)
(74) Representative: Grünecker Patent- und Rechtsanwälte PartG mbB
(86) International application number: PCT/JP2021/031710
(87) International publication number: WO 2023/031996

(57) **Abstract**

The present invention is to provide a pharmaceutical composition for suppressing invasion of cancer, which comprises siRNA that suppresses expression of CHST15 gene, and a method for suppressing invasion of cancer.

## Description

### TECHNICAL FIELD

The present invention relates to a pharmaceutical composition for suppressing progression of solid cancers and blood cancers, which comprises CHST15 siRNA as an effective ingredient. Proliferation of primary lesion alone cannot explain death by cancer, and the majority of deaths by cancer are considered to be the factors that are caused by invasion and metastasis, and further recurrence due to these. Invasion is the most important feature of malignancy of cancer and a major barrier to efficacy of the effects of cancer treatment (Documents 1 to 8). If the cancer is retained in the primary lesion, the effect of the anti-tumor lymphocytes by the immune checkpoint inhibitors will be more likely to be exerted, and in the solid cancers, it will be possible to completely cure by surgical resection. However, a treatment that actually suppresses invasion of cancer and retains the cancer in the primary lesion has not yet been clinically applied.

### BACKGROUND ART

Anticancer drugs in cancer drug therapy have heretofore been mainly therapeutic drugs that act on DNA replication and synthesis or protein synthesis, metabolic pathways, etc., and exert cell-killing effects by inhibiting cell proliferation and division. That is, side effects almost always occur because they exhibit cytotoxicity even to actively proliferating and dividing normal cells. Although the advent of molecular-targeted agents has led to more selective action against cancer cells and improved therapeutic outcomes, they have not yet achieved sufficient results, including side effects, and in most patients, reaction to first-line chemotherapy degrades and progress to second- or third-line chemotherapy (Documents 1 to 8). With the expectation of anticancer agents with a different mechanism of action from the conventional suppression of cell proliferation and division, immune checkpoint inhibitors, which kill cancer cells through the effect of the patient's own anti-tumor lymphocytes, have emerged and further improvement in therapeutic outcome has been recognized. However, the effect is still limited, and there is required for more multidisciplinary and comprehensive treatment with further anticancer agents.

Cancer death cannot be explained only by the proliferation of the primary lesion, and the majority of cancer deaths are considered to be caused by invasion, metastasis, and further subsequent recurrence. Invasion is the most prominent feature of malignancy of cancer and a major barrier to efficacy of cancer treatment (Documents 1 to 8) . If it is possible to retain the cancer at the primary lesion, the effect of the anti-tumor lymphocytes by the immune checkpoint inhibitors will be more likely to be exerted, and in the solid cancers, it will be possible to completely cure by surgical resection. However, a treatment that actually suppresses invasion of cancer and retains the cancer at the primary lesion has not yet been clinically applied.

CHST15 (Carbohydrate sulfotransferase 15), which is a glycosulfotransferase, is a type II transmembrane Golgi protein that transfers a sulfate group to the 6-position of the GalNAc (4SO₄) residue of chondroitin sulfate A (CS-A) to synthesize highly sulfated chondroitin sulfate E (CS-E) (Documents 9 and 10). It is rarely expressed in normal human tissues, but its expression is known to be enhanced in inflammation, fibrosis, and cancer. It has been reported that when expression of mRNA is suppressed by CHST15 siRNA, synthesis of CS-E is inhibited and fibrosis is suppressed in cardiac fibrosis, lung fibrosis, and gastrointestinal fibrosis diseases (Documents 11 to 16). In cancer, it has been reported that it suppresses cell proliferation of the human pancreatic cancer cell line Panc-1 *in vitro,* and in a mouse model of subcutaneously transplanted Panc-1, it also suppresses increase in tumor size, and is known to have anti-tumor effects by suppressing cancer cell proliferation (Document 17).

### PRIOR ART DOCUMENTS

### NON-PATENT DOCUMENTS

Non-Patent Document 1: Siegel RL, Miller KD, Fuchs HE, Jemal A. Cancer Statistics, 2021. CA Cancer J Clin 71:7-33, 2021.
Non-Patent Document 2: Falzone L, Salomone S, Libra M. Evolution of Cancer Pharmacological Treatments at the Turn of the Third Millennium. Front Pharmacol 9:1300, 2018.
Non-Patent Document 3: Murciano-Goroff YR, Warner AB, Wolchok JD. The future of cancer immunotherapy: microenvironment-targeting combinations. Cell Res 6:507-519, 2020.
Non-Patent Document 4: Hegde PS, Chen DS. Top 10 Challenges in Cancer Immunotherapy. Immunity 52:17-35, 2020.
Non-Patent Document 5: Waldman AD, Fritz JM, Lenardo MJ. A guide to cancer immunotherapy: from T cell basic science to clinical practice. Nat Rev Immunol 11:651-668,2020.
Non-Patent Document 6: Murciano-Goroff YR, Taylor BS, Hyman DM, Schram AM. Toward a More Precise Future for Oncology. Cancer Cell 37:431-442,2020.
Non-Patent Document 7: Fisher G, Rittie L. Restoration of the basement membrane after wounding: a hallmark of young human skin alters with aging. J Cell Commun Signal 12: 401-411, 2018.
Non-Patent Document 8: Martinez M, Moon EK. CAR T Cells for Solid Tumors: New Strategies for Finding, Infiltrating, and Surviving in the Tumor Microenvironment. Front Immunol 10:128, 2019.
Non-Patent Document 9: Ohtake S, Kondo S, Morisaki T, et al. Expression of sulfotransferase involved in the biosynthesis of chondroitin sulfate E in the bone marrow derived mast cells. Biochemical Biophysica Acta 1780: 687-95, 2008.
Non-Patent Document 10: Habuchi O, Moroi R, Ohtake S, et al. Enzymatic synthesis of chondroitin sulfate E by N-acetylgalactosamine 4-sulfate 6-O-sulfotransferase purified from squid cartilage. Anal Biochem 310: 129-36, 2002.
Non-Patent Document 11: Kai Y, Tomoda K, Yoneyama H et al. Silencing of carbohydrate sulfotransferase 15 hinders murine pulmonary fibrosis development. Mol Ther Nucleic Acid 6: 163-172, 2017.
Non-Patent Document 12: Sato H, Sagara S, Nakajima S, et al. Prevention of esophageal stricture after endoscopic submucosal dissection, using siRNA-based silencing of carbohydrate sulfotransferase 15 in pig. Endoscopy 49: 491-497, 2017.
Non-Patent Document 13: Suzuki K, Yokoyama J, Kawauchi Y, et al. Phase 1 clinical study of siRNA targeting carbohydrate sulfotransferase 15 in Crohn's disease patients with active mucosal lesions. J Crohns Colitis 11: 221-228, 2017.
Non-Patent Document 14: Suzuki K, Arumugam S, Yokoyama J, et al. Pivotal role of carbohydrate sulfotransferase 15 in fibrosis and mucosal healing in mouse colitis. PLoS One 11: e0158977, 2016.
Non-Patent Document 15: Watanabe K, Arumugam S, Sreedhar R, et al. Small interfering RNA therapy against carbohydrate sulfotransferase 15 inhibits cardiac remodeling in rats with dilated cardiomyopathy. Cell Signal 27: 1517-1524, 2015.
Non-Patent Document 16: Yamada S and Sugahara K. Potential therapeutic Application of chondroitin sulfate/dermatan sulfate. Current Drug Discovery Technologies 5: 289-301, 2008.
Non-Patent Document 17: Takakura K, Shibazaki Y, Yoneyama H, et al. Inhibition of Cell Proliferation and Growth of Pancreatic Cancer by Silencing of Carbohydrate Sulfotransferase 15 In Vitro and in a Xenograft Model. PLoS One 10:e0142981, 2015.

### SUMMARY OF THE INVENTION

### PROBLEMS TO BE SOLVED BY THE INVENTION

An object of the present invention is to provide a pharmaceutical composition (hereinafter also referred to as an invasion suppressing agent of cancer) which inhibits invasion of cancer cells and retains them from progressing in the solid cancers and blood cancers.

### MEANS TO SOLVE THE PROBLEMS

The present inventors investigated the effect of siRNA, which suppresses the expression of the CHST15 gene, using various human cancer cell lines, and found that it has an effect of suppressing the progress of cancer cells and retaining them localized, which is completely different from the effect of suppressing the proliferation of cancer cells predicted from conventional techniques. Also, they found that the effect thereof was also effective not only against solid cancer cells but also blood cancer cells. By retaining cancer cells localized through a mechanism different from proliferation control, it may be used in combination therapy to reduce the side effects of anticancer drugs and molecular-targeted drugs based on the conventional proliferation and division suppressing mechanism, in combination therapy with immune checkpoint inhibitors based on the anti-tumor immune mechanism, and further in the suppression of invasion and metastasis to enhance the efficacy of surgical operation and radiotherapy, etc., whereby the application to multidisciplinary cancer treatment and recurrence prevention was expected.

More specifically, the present invention is to provide the following [1] to [8].
[1] A pharmaceutical composition for suppressing invasion of cancer which comprises siRNA that suppresses expression of CHST15 gene.
[2] The pharmaceutical composition described in [1] for suppressing metastasis of cancer.
[3] The pharmaceutical composition described in [1] or [2], wherein the cancer is a solid cancer or a blood cancer.
[4] The pharmaceutical composition described in any of [1] to [3], characterized in that the progress of the cancer is suppressed by retaining the cancer.
[5] A method for suppressing invasion of cancer, which comprises a step of administering siRNA that suppresses expression of CHST15 gene.
[6] An siRNA that suppresses expression of CHST15 gene for use in a method for suppressing invasion of cancer.
[7] Use of an siRNA that suppresses expression of CHST15 gene in the production of an invasion suppressing agent of cancer.
[8] A method for producing an invasion suppressing agent of cancer, which comprises a step of using siRNA that suppresses expression of CHST15 gene.

### EFFECTS OF THE INVENTION

In the progression of cancer cells, there are processes of proliferation at the primary lesion, invasion, metastasis, and angiogenesis at metastatic lesion, each of which is regulated by different mechanisms. Most of the existing anticancer agents and molecular targeted drugs mainly suppress proliferation, but there is a problem of side effects due to their effects on normal cells as well. In addition, due to the Dichotomy (choice of either proliferation or migration) of cell proliferation and migration, proliferation is suppressed and migration can be inversely activated, which is said to be a factor against anticancer agents and radiotherapy, in particular, in cancer cells with high migration capacity. Therefore, in the CHST15 siRNA, there was a concern about a possibility that it activates migration inversely, considering the previously reported suppressing effect on proliferation.

Thus, when *in vitro* test was carried out using BxPC-3 cells, which are pancreatic cancer similar to the previously reported Panc-1 cells but are a different cell line, almost no proliferation suppressing effect was admitted by suppressing expression of CHST15 by adding CHST15 siRNA, but an unexpected result that invasion was suppressed was obtained. Furthermore, surprisingly, it was revealed for the first time that this invasion suppressing effect is present not only against solid cancers but also against blood cancers.

According to the present invention, it can be applied to multidisciplinary cancer treatment and recurrence prevention, such as combination therapy that can reduce the side effects of conventional anticancer agents and molecular-targeted drugs based on the conventional proliferation and mitogenic inhibitory mechanisms, combination therapy with immune checkpoint inhibitors based on anti-tumor immune mechanisms, and further to enhance the effect of surgical operation and radiotherapy by suppressing invasion and metastasis.

### BRIEF DESCRIPTION OF THE DRAWINGS

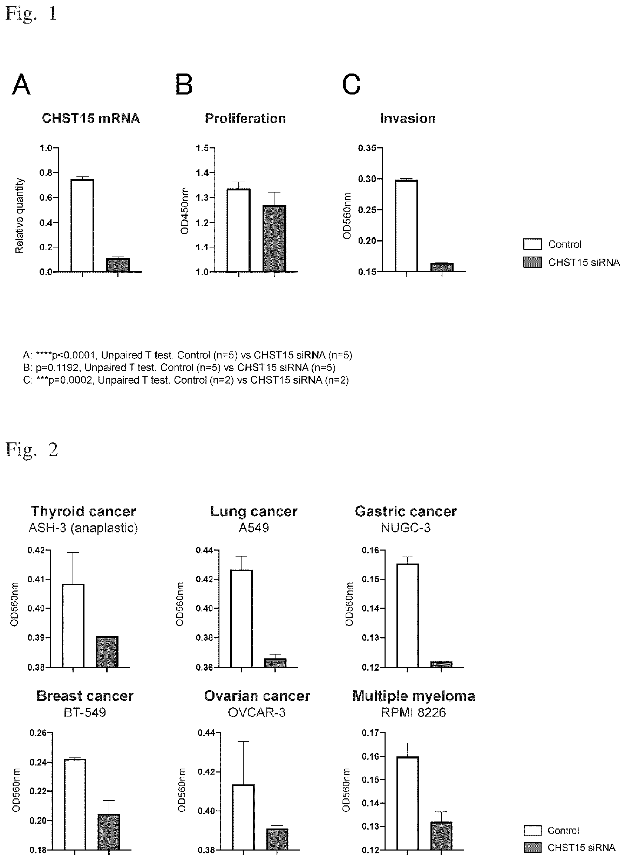

Fig. 1 shows a knockdown effect by CHST15 siRNA (A), proliferation suppressing effect (B), and invasion suppressing effect (C) in the human pancreatic cancer cell line BxPC-3.
Fig. 2 shows an invasion suppressing effect in various kinds of human cancer cell lines by CHST15 siRNA. In addition to the solid cancers such as human thyroid undifferentiated carcinoma cell line ASH-3, human lung cancer cell line A549, human breast cancer cell line BT-549, human stomach cancer cell line NUGC-3, and human ovarian cancer cell line OVCAR-3, etc., invasion suppression was also observed in human multiple myeloma cancer cell line RPMI8226.

### EMBODIMENTS TO CARRY OUT THE INVENTION

Hereinafter, the present invention will be explained in detail.

The present inventor has found that the invasion suppressing effect was expressed not only of solid cancers but also of blood cancers by suppressing expression of CHST15 (Carbohydrate sulfotransferase 15) gene. More specifically, the present inventor has found that the invasion suppressing effect was expressed not only of solid cancers but also of blood cancers by suppressing expression of CHST15 gene by an RNAi (RNA interference; RNA interference) effect.

The CHST15 gene of the present invention is not particularly limited, and it is generally derived from an animals, more preferably derived from mammals, and most preferably derived from human. Incidentally, the CHST15 of the present invention is also called to as GalNAc4S-6ST (N-acetylgalactosamine 4-sulfate 6-0 sulfotransferase) as another name.

The sequence of CHST15 (GalNAc4S-6ST) of the present invention can be obtained, for example, based on accession number NM_015892. As an example, the base sequence of the CHST15 gene of the present invention is shown in SEQ ID NO: 3, and the amino acid sequence encoded by the gene is shown in SEQ ID NO: 4. Even if the protein consists of an amino acid sequence other than the above, for example, a protein having a high identity (generally 70% or more, preferably 80% or more, more preferably 90% or more, and most preferably 95% or more) with the sequence shown in SEQ ID NO: 4, and having the functions of the above-mentioned protein is included in the CHST15 protein of the present invention. The above-mentioned protein means, for example, a protein consisting of an amino acid sequence in which one or more amino acids are added, deleted, substituted, or inserted in the amino acid sequence shown in SEQ ID NO: 4, and the number of amino acids that are generally changed is within 30 amino acids, preferably within 10 amino acids, more preferably within 5 amino acids, and most preferably within 3 amino acids.

In the above-mentioned gene of the present invention, for example, endogenous genes in other organisms (homologs of the above-mentioned gene in humans, etc.) corresponding to DNA consisting of the base sequence shown in SEQ ID NO: 3 are contained. In addition, the endogenous DNA in other organisms corresponding to DNA consisting of the base sequence shown in SEQ ID NO: 3 generally has high identity (homology) with the DNA shown in SEQ ID NO: 3, respectively. High identity means homology of preferably 70% or more, further preferably 80% or more, and more preferably 90% or more (for example, 95% or more, further 96%, 97%, 98% or 99% or more). This homology can be determined by the mBLAST algorithm (Altschul et al. (1990) Proc. Natl. Acad. Sci. USA 87: 2264-8; Karlin and Altschul (1993) Proc. Natl. Acad. Sci. USA 90: 5873-7). Also, when the DNAs are isolated from living organisms, they can be considered to hybridize under stringent conditions with the DNAs shown in SEQ ID NO: 3, respectively. Here, the "stringent conditions" may be mentioned, for example, "2 x SSC, 0.1% SDS, 50°C", "2 x SSC, 0.1% SDS, 42°C", "1 x SSC, 0.1% SDS, 37°C", and as more stringent conditions, there may be mentioned the conditions of "2 x SSC, 0.1% SDS, 65°C", "0.5 x SSC, 0.1% SDS, 42°C" and "0.2 x SSC, 0.1% SDS, 65°C".

In the present specification, "siRNA that suppresses expression of the CHST15 gene" can also be expressed as "CHST15 siRNA", which is preferably siRNA with a structure having bases overhung at the 3' end of one strand and at the 3' end of the other strand, and more preferably siRNA with a structure in which the RNAs shown in SEQ ID NO: 1 and 2 are hybridized.

The siRNA in the present invention does not necessarily have to have all of its nucleotides as ribonucleotides (RNA). That is, in the present invention, one or a plural number of ribonucleotides constituting siRNA may be corresponding deoxyribonucleotides if the molecule itself has the function of suppressing expression of the CHST15 gene. This "corresponding" refers to the same base species (adenine, guanine, cytosine, thymine (uracil)), although the structure of the sugar moiety is different. For example, a deoxyribonucleotide corresponding to a ribonucleotide having adenine refers to a deoxyribonucleotide having adenine. In addition, the above-mentioned "a plural number" is not particularly limited, and refers to a small number of preferably about 2 to 5.

The siRNA of the present invention can be appropriately made by a person skilled in the art using a commercially available nucleic acid synthesizer. Also, it is possible to use general contract synthesis services for synthesis of the desired RNA.

The CHST15 siRNA of the present invention has an effect of suppressing invasion of cancer or retaining cancer as a single agent, so that the present invention provides an agent for suppressing invasion of cancer containing CHST15 siRNA an effective ingredient, that is, a pharmaceutical composition for treatment or prevention of suppressing invasion of cancer. Alternatively, the present invention provides a method for suppressing invasion of cancer which comprises a step of administering siRNA that suppresses expression of CHST15 gene, siRNA that suppresses expression of CHST15 gene to be used in the method for suppressing invasion of cancer, use of siRNA that suppresses expression of CHST15 gene in the manufacture of a restorative, and a method for producing an agent for suppressing invasion of cancer which comprises a step of using siRNA that suppresses expression of CHST15 (formulating and/or mixing with a pharmaceutically or physiologically acceptable carrier).

Cancers which become objects to be treated or prevented in the present invention are not particularly limited as long as they are solid cancers and blood cancers for which siRNA of the present invention that inhibits the expression of the CHST15 gene has a therapeutic effect, and include cancers or tumors of breast, heart, lung, small intestine, colon, spleen, kidney, bladder, head, neck, ovary, prostate, brain, pancreas, skin, bone, bone marrow, blood, thymus, uterus, testis and liver. In a specific embodiment, cancers which become objects to be treated or prevented in the present invention include tumors, which are adenomas, adenocarcinomas, angiosarcomas, astrocytomas, epithelial carcinomas, germ cell tumors, glioblastomas, gliomas, angioendotheliomas, hemangiosarcomas, hematomas, hepatoblastomas, leukemias, lymphomas, medulloblastomas, melanomas, neuroblastomas, osteosarcomas, retinoblastomas, rhabdomyosarcomas, sarcomas, teratoma, acral lentiginous melanoma, solar keratosis, adenocarcinoma, adenoid cystic carcinoma, adenoma, adenosarcoma, adenosquamous carcinoma, astrocytoma, Bartholin's adenocarcinoma, basal cell carcinoma, bronchial adenocarcinoma, capillary carcinoid, cell carcinoma, carcinosarcoma, cholangiocellular carcinoma, chondrosarcoma, cystadenoma, yolk sac tumor, endometrial hyperplasia, endometrial stromal sarcoma, endometrioid adenocarcinoma, ependymoma, Ewing's sarcoma, localized nodular hyperplasia, gastrin-producing tumor, germ line tumor, glioma, glucagon-producing tumor, hemangioblastoma, hemangioendothelioma, hemangioma, hepatic adenoma, hepatic adenomatosis, hepatocellular carcinoma, insulinite, epithelial tumor, intraepithelial squamous cell tumor, invasive squamous cell carcinoma, large cell carcinoma, liposarcoma, lung cancer, lymphoblastic leukemia, lymphocytic leukemia, smooth muscle tumor, melanoma, malignant melanoma, malignant mesothelioma, neurolemmoma, medulloblastoma, medulloepithelioma, mesothelioma, mucoepidermoid carcinoma, myeloid leukemia, neuroblastoma, neuroepithelial adenocarcinoma, nodular melanoma, osteosarcoma, ovarian cancer, serous papillary adenocarcinoma, pituitary tumor, plasmacytoma, pseud sarcoma, prostate cancer, lung blastoma, renal cell carcinoma, retinoblastoma, rhabdomyosarcoma, sarcoma, serous carcinoma, squamous cell carcinoma, small cell carcinoma, soft tissue carcinoma, somatostatin-secreting tumor, squamous cell carcinoma, squamous cell carcinoma, undifferentiated carcinoma, uveal melanoma, verrucous carcinoma, vaginal/vulvar cancer, VIP (vasoactive intestinal peptide) producing tumor, and Wilms tumor, brain cancer, head and neck cancer, colorectal cancer, acute myelogenous leukemia, precursor B-cell acute lymphoblastic leukemia, bladder cancer and astrocytoma, preferably grade II, III, or IV astrocytoma, glioblastoma, glioblastoma multiforme, small cell carcinoma, and non-small cell carcinoma, preferably non-small cell lung cancer, lung adenocarcinoma, metastatic melanoma, androgen-independent metastatic prostate cancer, androgen-dependent metastatic prostate cancer, prostate adenocarcinoma, breast cancer, ductal carcinoma, and particularly preferably glioblastoma, pancreatic cancer, breast cancer, bladder cancer, kidney cancer, head and neck cancer, ovarian cancer, colon cancer, cervical cancer, prostate cancer, lung cancer, stomach cancer, thyroid cancer, or plasmacytic myeloma.

In the present invention, "suppressing invasion of cancer" means to retain cancer cells at the primary lesion, to prevent progression and metastasis of cancer, and/or to enhance therapeutic effects of cancer. Incidentally, in the "treatment" in the present invention, it is not necessarily limited to the case of having a complete therapeutic effect, and may be the case of having a partial effect.

The pharmaceutical composition for suppressing invasion of cancer of the present invention can be administered orally or parenterally after being mixed with a pharmaceutically or physiologically acceptable carrier, excipient or diluent. As the oral preparations, it can be made dosage forms such as granules, powders, tablets, capsules, solutions, emulsions or suspensions, etc. As the parenteral agent, it can be selected dosage forms such as injections, infusions, drugs for external use, inhalants (nebulizers) or suppositories, etc. As the injections, there may be shown subcutaneous injections, intramuscular injections, intraperitoneal injections, intracranial injections or intranasal injections, etc. As the drugs for external use, there may be shown transnasal medicines or ointments, etc. Techniques for preparing the above-mentioned dosage forms so as to contain the pharmaceutical composition of the present invention, which is the main ingredient, are known.

For example, tablets for oral administration can be prepared by mixing the pharmaceutical composition for restoring tissue integrity of the present invention with excipients, disintegrants, binders and lubricants, etc., and then compressing and shaping the mixture. As the excipients, lactose, starch or mannitol, etc., is generally used. As the disintegrants, calcium carbonate, carboxymethylcellulose calcium, etc., are generally used. As the binders, gum arabic, carboxymethylcellulose or polyvinylpyrrolidone is used. As the lubricants, talc and magnesium stearate are well known.

The tablets containing the pharmaceutical composition for suppressing invasion of cancer of the present invention can be applied thereto masking or known coatings to make the formulation enteric soluble. As the coating agent, ethyl cellulose or polyoxyethylene glycol, etc., can be used.

Also, the injections can be obtained by dissolving the pharmaceutical composition for suppressing invasion of cancer of the present invention, which is the main ingredient, together with a suitable dispersant, or dissolving or dispersing the same in a dispersant. Depending on the selection of the dispersant, the formulation can be made either of the dosage form of an aqueous solvent and an oily solvent. For making the aqueous solvent, distilled water, physiological saline solution or Ringer's solution, etc., is used as a dispersant. In the oily solvent, various kinds of vegetable oils or propylene glycol, etc., is utilized as a dispersant. At this time, preservatives such as parabens can be added as necessary. Also, in the injections, known tonicity agents such as sodium chloride or glucose, etc., can be added. Further, soothing agents such as benzalkonium chloride and procaine hydrochloride can be added.

In addition, the pharmaceutical composition for suppressing invasion of cancer of the present invention can be made into a topical application by making it into a solid, liquid or semi-solid composition with or without excipients or carriers. Regarding the solid or liquid composition, they can be made into a topical application by making a composition similar to those described above. The semi-solid composition can be prepared by adding a thickening agent as needed to an appropriate solvent. As the solvent, water, ethyl alcohol or polyethylene glycol, etc., can be used. As the thickening agent, bentonite, polyvinyl alcohol, acrylic acid, methacrylic acid or polyvinyl pyrrolidone, etc., is generally used. To the composition, preservatives such as benzalkonium chloride, etc., can be added. Also, it can be made into a suppository by combining an oil base material such as cocoa fat or an aqueous gel base material such as a cellulose derivative, as a carrier.

When the pharmaceutical composition for suppressing invasion of cancer of the present invention is used as a gene therapeutic agent, there may be mentioned a method of administering a vector in which the nucleic acid is incorporated in addition to a method of directly administering the invasion suppressing agent of cancer or the pharmaceutical composition of the present invention by injection. As the above-mentioned vector, there may be mentioned adenovirus vector, adeno-associated virus vector, herpes virus vector, vaccinia virus vector, retrovirus vector, lentivirus vector, etc., and by using these virus vectors, they can be efficiently administered.

In addition, it is possible to introduce the pharmaceutical composition for suppressing invasion of cancer of the present invention into phospholipid vesicles such as liposomes and administer the vesicles. siRNA-retained vesicles are introduced into a predetermined cell by lipofection method. And, the resulting cells are, for example, systemically administered intravenously or intra-arterially, etc.

In addition, the present invention provides a method for suppressing invasion of cancer in a subject, which comprises a step of administering the pharmaceutical composition for suppressing invasion of cancer of the present invention to an individual (for example, a patient) or tumor tissue thereof. The individual that becomes a subject of the present invention is not particularly limited as long as it is an organism that can develop a tumor, and preferably a human. Administration to the subject can be carried out, for example, by a method known to a person skilled in the art such as oral administration, intratumoral administration, intradermal administration or subcutaneous administration, or intravenous injection, etc. It is possible to carry out systemic administration or direct local administration into the tumor tissue. Also, commercially available gene transfer kits can also be used to introduce the siRNA of the present invention into target cells, tissues or organs.

The pharmaceutical composition for suppressing invasion of cancer of the present invention is administered to mammalian subjects, including humans, in necessary (effective) amounts within the dosage range considered to be safe. A dosage of the pharmaceutical composition for suppressing invasion of cancer of the present invention can be ultimately determined by a person skilled in the art (physician or veterinarian) in consideration of the type of dosage form, method of administration, age and weight of the subject, symptoms of the subject, etc. To give an example, although it varies depending on age, sex, symptoms, route of administration, frequency of administration and dosage form, for example, the dosage for topical administration of CHST15 siRNA of the present invention is about 1 pM to 1 mM per day once, and is administered singly or at weekly intervals to monthly intervals (for example, at weekly intervals, 2-week intervals or 1-month intervals), or administered daily.

Incidentally, all prior art documents cited in the present specification are hereby incorporated into the present specification by reference.

### EXAMPLES

Hereinafter, the present invention will be more specifically explained by referring to Examples. However, the technical scope of the present invention is not limited to these examples.

Incidentally, "CHST15 siRNA" used in this Examples is an siRNA with a structure in which RNAs described in SEQ ID NO: 1 and 2 are hybridized. Regarding the base sequence of the GalNac 4S-6ST siRNA drug used in the present Example, it is shown below. The sequence is not limited in this example alone. [human GalNac4-6STsiRNA] (Gene Bank accession number NM_015892) (available from Hokkaido System Science Co., Ltd.)
5'-ggagcagagcaagaugaauacaauc-ag-3' (SEQ ID NO: 1)
3'-ua-ccucgucucguucuacuuauguuag-5' (SEQ ID NO: 2)

### <Example 1>

Panc-1 cells, which are known to have a cell proliferation suppressing effect in prior art reference, are human pancreatic cancer cell lines, so that BxPC-3 cells, which are also human pancreatic cancer cell lines, were used. Higher expression of COX-2, PGE2 and VEGF was reported in BxPC-3 cell lines as compared to Panc-1 cells, and the possibility of difference in angiogenic potential is suggested, but regarding proliferation potential and invasive potential, no significant differences have been reported between both cells (review article: Deer EL, et al. Phenotype and genotype of pancreatic cancer cell lines. Pancreas. 39:425-435, 2010).

In the *in vitro* tests, BxPC-3 cells were cultured according to standard methods. In 24 well plates, CHST15 siRNA was transfected by adjusting a density of 0.5 × 10⁵ cells per each well. Transfections were carried out using a Lipofectamine RNAiMAX reagent (available from Thermo Fisher Scientific Inc.), adjusting a concentration of siRNA to be 50 nM or 500 nM and culturing under humidified conditions at 37°C for 48 hours at a CO₂ concentration of 5%. Incidentally, as a negative control, a vehicle (physiological saline) was used. After culturing for 48 hours, total RNA was extracted from the cells in each well of the 24 well plate, and cDNA was synthesized using 0.1 µg of the total RNA. The synthesized cDNA was diluted with DNase/RNase-Free Water so as to be 1 ng/µL (template amount was 5 ng/reaction), and expression of CHST15 mRNA was measured using the qPCR method. The results are shown in Fig. 1A. 50 nM CHST15 siRNA markedly suppressed CHST15 mRNA in BxPC-3 cells.

On the other hand, the cells after transfection were collected and their proliferation potential and invasive potential were evaluated. For cell proliferation, cells were cultured at a density of 1.0 × 10⁴ to 1.0 × 10⁶ Cells/mL under humidified conditions at 37°C for 24 hours at a CO₂ concentration of 5%, followed by using Cell Counting Kit-8 (manufactured by DOJINDO Chemical Research) and measuring at OD450 nM using a plate reader. The results are shown in Fig. 1B. 500 nM CHST15 siRNA had no effect on proliferation of BxPC-3 cells.

For cell invasion, after adjusting a density to 0.2 × 10⁵ Cells/300 µL, a cell invasion test was carried out using CytoSelect 24-well Cell Invasion Assay Kit (manufactured by CELL BIOLABS). After culturing for 24 hours and 48 hours, and after staining the invaded cells, it was measured at OD560 nM using a plate reader. The results are shown in Fig. 1C. 50 nM CHST15 siRNA markedly suppressed invasion of BxPC-3 cells. This indicates that there is an effect of retaining cancer cells in the well to which they have been added.

### <Example 2>

By the same method as in Example 1, CHST15 siRNA was transfected using various human cancer cell lines, and Cell Invasion Assay was carried out. Human cancer cells were analyzed not only solid cancers but also blood cancers, including A549 (lung cancer), NUGC-3 (stomach cancer), ASH-3 (undifferentiated thyroid cancer), BT-549 (breast cancer), OVCAR-3 (ovarian cancer) and RPMI8226 (plasmacytic myeloma). The results are shown in Fig. 2. 50 nM CHST15 siRNA clearly suppressed invasion of all kinds of human cancer cells carried out.

### Consideration:

CHST15 siRNA suppressed invasion of not only human solid cancer cells but also human blood cancer cells. By retaining the cancer cells, it was suggested to suppress progression of cancer.

### UTILIZABILITY IN INDUSTRY

The CHST15 siRNA of the present invention significantly suppressed invasion of human cancer cell lines, and the effect was observed not only in solid cancers but also unexpectedly in blood cancers. By retaining the cancer cells at the primary cite (primary solid organs in solid cancers, and bone marrow or lymphoid organs in blood cancers), it was thought that new cancer therapeutic effects could be derived.

From these results, it was shown that the siRNA that suppresses expression of CHST15 gene of the present invention was useful for suppressing invasion of cancer.

### Sequence Listing

FP4713PCT ST25.txt

## Claims

1. A pharmaceutical composition for suppressing invasion of cancer which comprises siRNA that suppresses expression of CHST15 gene.

2. The pharmaceutical composition according to Claim 1, which is for suppressing metastasis of cancer.

3. The pharmaceutical composition according to Claim 1 or 2, wherein the cancer is a solid cancer or a blood cancer.

4. The pharmaceutical composition according to any one of Claims 1 to 3, **characterized in that** it inhibits progression by retaining the cancer.

5. A method for inhibiting invasion of cancer, which comprises a step of administering siRNA that suppresses expression of CHST15 gene.

6. An siRNA that suppresses expression of CHST15 gene for use in production of an invasion suppressing agent of cancer.

7. Use of an siRNA that suppresses expression of CHST15 gene in the production of an invasion suppressing agent of cancer.

8. A method for producing an invasion suppressing agent of cancer, which comprises a step of using siRNA that suppresses expression of CHST15 gene.
